# EUROPEAN PATENT APPLICATION

(11) **EP 3 372 204 A1**
(43) Date of publication of application: **12.09.2018**
(21) Application number: 17160450.7
(22) Date of filing: 10.03.2017
(51) Int. Cl.: A61F 7/00, A61F 7/12, A61M 1/36

(54) **WHOLE-BODY HYPERTHERMIA SYSTEM**

(71) Applicant: ELMEDIX BVBA, 3001 Leuven (BE)
(72) Inventor: Van den Bossche, Johan, B-3210 Linden (BE); Conings, Liesbet, B-3210 Linden (BE); Bogers, John-Paul, B-2530 Boechout (BE)
(74) Representative: IPLodge bvba

(57) **Abstract**

The invention relates to whole-body hyperthermia systems for raising the body temperature of the entire body of an individual to a predetermined target temperature Tb and for stabilizing said body temperature at said target temperature Tb, said systems comprising an apparatus having a first thermal means for raising said body temperature at a rate of at least 1 °C/hour, and at least a second thermal means for further adjusting the body temperature to said target temperature Tb, wherein said second thermal means comprise a fluid circuit, which is at least partially within the individual, wherein a fluid, heated by a heat exchanger to a set temperature Tf, is circulated through said fluid circuit.

## Description

### Technical Field

The present invention relates to a whole-body hyperthermia system for raising the body temperature of an individual to a predetermined target temperature T_{b} and for stabilizing the body temperature at the target temperature T_{b}. The present invention further relates to a method for subjecting a patient to a whole-body hyperthermia condition and to an anti-tumour compound for use in the treatment of e.g. cancer.

### Background Art

Cancer therapies aim to maximally remove tumour tissue while preserving healthy tissue as much as possible. However, surgery and irradiation are locally applied, and a small amount of cancer cells which are at remote positions from the site of intervention are not detected or removed. Equally, chemotherapy often does not kill all tumour cells.

Consequently, alternative anti-tumour therapies are needed.

It has been described that tumour cells are sensitive for stress conditions, such as elevated temperatures. Indeed, treatment at temperatures between 40 and 44 °C is cytotoxic for cells in an environment with a low pO(2) and low pH, conditions that are found specifically within tumour tissue, due to insufficient blood perfusion. Under such conditions radiotherapy is less effective, and systemically applied cytotoxic agents will reach such areas in lower concentrations than in well perfused areas. Therefore, the addition of hyperthermia to radiotherapy or chemotherapy will result in at least an additive effect. Furthermore, the effects of both radiotherapy and many drugs are enhanced at an increased temperature. Hyperthermia can be applied by several methods: local hyperthermia by external or internal energy sources, regional hyperthermia by perfusion of organs or limbs, or by irrigation of body cavities, and whole-body hyperthermia. [reviewed in van der Zee (2002) Ann. Oncol. 13, 1173-1184; Heating the patient: a promising approach?].

Cihoric N et al. (2015) Int J Hyperthermia 31, 609-614 "Hyperthermia-related clinical trials on cancer treatment within the ClinicalTrials.gov registry." gives an overview of 109 clinical trials in which hyperthermia was part of the treatment regimen. 12 of these were performed with whole body hyperthermia. Devices and methods to apply local hyperthermia have been described in EP2401033, EP2401033, EP2284413, EP1581145.

Worel et al. (2014) Int. J. Artif. Organs. 37, 1-12 describe methods and devices to apply whole body hyperthermia on cancer patients under moderate sedation enabling spontaneous breathing.

Elevated temperatures, mimicking severe near-lethal fever, are difficult to apply in a "whole-body" approach, especially for prolonged periods (e.g. more than 6 hours).

Methods and devices allowing a long term whole body hyperthermia with minimal discomfort and high efficacy are lacking.

### Summary of the invention

The methods and devices of the present invention has the advantages that whole body hyperthermia can be applied for much longer time periods than used in the prior art.

Prior art methods are applied on patients for short periods because of the discomfort of the patient which is at most moderately sedated. The methods and devices of the present invention allow to compensate for the discomfort that occurs when the whole body temperature is applied for more than 4 h, more than 6 h, or more than 12 h.

The methods of the present invention have the advantage that tumour cells are challenged for a much longer time and that tumour cells death occurs more efficient compared to shorter treatments.

The methods of the present invention avoid the selection of heat-resistant tumour cells as may occur with repetitive exposure to hyperthermic conditions for shorter time periods. Indeed, sub-optimal conditions as used in the prior art may select those cells with superior heat shock protein repair systems, making the cells resistant for further hyperthermic conditions. With the methods of the present invention, the risk of selecting heat resistant cells is dramatically reduced.

This consequently allows repeating long term hypothermic treatments, especially in combination with chemotherapy. Indeed, high temperatures improve the uptake of anti-cancer drugs allowing the use of lower doses of medicaments, and consequently fewer side effects. The methods of the present invention allow repetitive treatments of long term hyperthermia combined with chemotherapy at reduced dosages. In combination treatment the chemotherapeutic can be applied before, during or after the hyperthermia treatment.

The present invention has the advantage that heat loss from the body is reduced and kept to its minimum. When extracorporeal heating devices are used, the temperature at the heating device can be kept close to the target temperature to be achieved at the body. Prior art methods and devices wherein an individual is wrapped in blanket loose substantial heat via the skin, or via breathing, such that the heating devices of the patient have to compensate the heat loss by heating the blood to temperatures above the target temperature of the body.

The different measures for minimising the loss of heat from the body have the advantages that the excess of heat that has to be applied to the body is as low as possible. This in contrast with the prior art systems with extracorporeal blood heating wherein the blood needs to be heated to substantially higher temperatures than the target temperature to compensate for loss of e.g. heat by respiration, or inadequate isolation of the body.

One aspect of the invention relates to whole-body hyperthermia systems for raising the body temperature of the entire body of an individual to a predetermined target temperature Tb and for stabilizing the body temperature at the target temperature Tb, systems comprising:
- a cabinet with isolation and heating for maintaining an individual at an elevated temperature,
- at least one thermal means for raising and stabilising the temperature of the individual,
- one or more devices to prevent or counter heat loss from the individual,
- one or more sensors monitoring the temperature or heat flux of the individual, connected to controllers steering the devices to prevent or counter heat loss and steering the at least one thermal means.

Herein the devices to prevent or counter heat loss are typically devices for heating air and/or oxygen for respiration, or devices for heating liquids being introduced in the body.

In embodiments of these systems the sensor monitoring the temperature of the individual are sensors measuring the temperature internally, sensors measuring the temperature externally and contacting the skin, or sensors measuring the temperature externally and not contacting the skin.

In embodiments of these systems, the sensor monitoring the temperature of the individual are one or more heat flux sensors attached to the skin of the individual.

Embodiments of these systems, comprise a first thermal means for raising the body temperature at a rate of at least 1 °C/hour, and at least a second thermal means for further adjusting the body temperature to the target temperature Tb,
wherein the second thermal means comprise a fluid circuit, which is at least partially within the individual, wherein a fluid, heated by a heat exchanger to a set temperature Tf, is circulated through the fluid circuit.

In embodiments of these systems, the first and the second means are different from each other.

In embodiments of these systems, the fluid circuit is an internally heated fluid circuit.

In embodiments of these systems the fluid circuit is an internally heated catheter.

In embodiments of these systems, the first thermal means comprises a pump selected from the group consisting of a roller pump, a centrifugal pump, a pulsatile pump and a non-occlusive roller pump.

In embodiments of these systems, the system is provided with means for monitoring the body temperature and/or vital signs of the individual, such as respiratory activity, and/or cardiac activity and blood parameters.

In embodiments of these systems, the means for monitoring the body temperature is a heat sensitive camera measuring the temperature of a part of the skin of the body.

In embodiments of these systems, the means for monitoring the body temperature comprise at least one temperature probe for monitoring at least one temperature signal at at least one location on or within the individual; and means for deriving an overall value, being the monitored body temperature Tm, from the at least one temperature signal.

In embodiments of these systems, a controller uses at least the monitored body temperature Tm for generating a control signal for the first and second thermal means for steering Tm to the target temperature Tb.

In embodiments of these systems the system is provided with means for maintaining the monitored body temperature Tm within a range of at most 0.5 °C from the predetermined target temperature Tb.

In embodiments of these systems, the system is provided with means to bring the individual under sedation or complete narcosis, and with means to supply oxygen to the individual, wherein the oxygen supplying means are provided to supply oxygen to the individual at a temperature Tb.

In embodiments of these systems, the first and second thermal means are provided for raising the body temperature to the target temperature Tb for maintaining Tb at least a period of time tp, wherein the period of time tp is at least 6 hours, at least 8 hours or at least 12 hours.

In embodiments of these systems, the system is provided with means to administer additional compounds to the individual, such as transfused blood, chemotherapeutic drugs and/or nutritional compounds, such as salt solutions, and wherein the additional compounds are administered at a temperature Tb.

In embodiments of these systems, the system is provided with one or more isolation elements for an individual, such as isolation foil or suit for torso or limbs, or a helmet or cap like device for application on the head.

In embodiments of these systems the first thermal means has at least a first portion and at least a second portion, wherein the at least first portion of the first thermal means is arranged to raise the body temperature of at least a first series of body parts of the individual to the target temperature Tb, and wherein the at least second portion of the first thermal means is arranged to prevent at least a second series of body parts to exceed a target temperature Ts, wherein the temperature Ts is lower than the temperature Tb.

Another aspect of the invention relates to disposable kits for use in the above systems, comprising tubing for a fluid circuit for blood supply to an individual and comprising sensors to monitor the temperature or heat loss from the patient.

Such kits may further comprise an isolation element for an individual, such as isolation foil or suit for torso or limbs, or a helmet or cap like device for application on the head.

Another aspect of the invention relates to methods for subjecting an individual to a whole-body hyperthermia condition using a whole-body hyperthermia system as described above, the method comprising the steps of
- placing the individual in a heated cabinet;
- applying means for monitoring the body temperature;
- deriving from the monitoring of the body temperature an overall value, being the monitored body temperature Tm;
- generating a control signal from the monitored body temperature Tm for thermal means, for steering Tm to the target temperature Tb;
whereby the individual is kept in hyperthermia condition for at least 6 hours, or at least 8 hours, or at least 12 hours, during which period the monitored body temperature Tm of the patient does not deviate by more than 0.5 °C, or 1,0°C from the predetermined target temperature Tb.

In embodiments of these methods, heat losses from the individual are compensated by one or more of the following steps:
- heating the skin of the individual or parts thereof by controlling the temperature of the air surrounding the individual;
- applying isolation on one or more parts of the body;
- heating air used for respiration to a controlled temperature;
- heating liquids for administering, nutrients, moisture or medicaments to a controlled temperature.

In embodiments of these methods, the first and second thermal means are used which differ from each other.

In embodiments of these methods, the individual is kept in hyperthermia condition at a temperature between 40 and 42 °C.

In embodiments of these methods, wherein the individual is kept in hyperthermia condition for at least 12 hours.

In typical embodiment of these methods, the individual is a cancer patient.

In typical embodiment of these method, the individual is under complete narcosis.

Another aspect relates to the use of a kit in accordance as described above, in a method as described before.

Another aspect relates to an antitumor compound for use in the treatment of cancer, wherein the compound is administered to a patient which is under a whole body hyperthermia of between 41.5 °C and 42.0 °C for at least 6 h.

One aspect of the invention relates to whole-body hyperthermia systems for raising the body temperature of the entire body of an individual to a predetermined target temperature Tb and for stabilizing the body temperature at the target temperature Tb, comprising an apparatus having a first thermal means for raising the body temperature at a rate of at least 1 °C/hour, and at least a second thermal means for further adjusting the body temperature to the target temperature Tb, wherein the second thermal means comprise a fluid circuit, which is at least partially within the individual, wherein a fluid, heated by a heat exchanger to a set temperature Tf, is circulated through the fluid circuit.

In embodiments thereof, the first and the second means are different from each other.

In embodiments thereof, the first and the second means are the same.

In embodiments thereof, the fluid circuit is an internally heated fluid circuit, typically an internally heated catheter.

In embodiments thereof first thermal means comprise a pump selected from the group consisting of a roller pump, a centrifugal pump, a pulsatile pump and a non-occlusive roller pump.

In embodiments thereof the systems are provided with means for monitoring the body temperature and/or vital signs of the individual, such as respiratory activity and/or cardiac activity.

In embodiments thereof means for monitoring the body temperature is a heat sensitive camera measuring the temperature of a part of the skin of the body.

In embodiments thereof, means for monitoring the body temperature comprise at least one temperature probe for monitoring at least one temperature signal at at least one location on or within the individual; and means for deriving an overall value, being the monitored body temperature Tm, from the at least one temperature signal.

The monitoring of the body temperature refers to thermometers measuring the temperature (internal, external, remote, heat map) or flux sensors measure the heat loss using applied on the body. Flux sensors have the advantages that the flux is determined over the entire surface of the sensor and provide a more accurate measurement than local temperature determination of a thermometer.

In embodiments thereof, a controller uses at least the monitored body temperature Tm for generating a control signal for the first and second thermal means for steering Tm to the target temperature Tb.

In embodiments thereof, the system is provided with means for maintaining the monitored body temperature Tm within a range of at most 0.5 °C, preferably at most 0,1°C from the predetermined target temperature Tb.

In embodiments thereof, the system is provided with means to bring the individual under sedation, and with means to supply oxygen to the individual, wherein the oxygen supplying means are provided to supply oxygen to the individual at a temperature Tb.

In embodiments thereof, first and second thermal means are provided for raising the body temperature to the target temperature Tb for at least a period of time tp, wherein the period of time tp is at least 6 hours, at least 8 hours or at least 12 hours.

In embodiments thereof the system is provided with means to administer additional compounds to the individual, such as transfused blood, chemotherapeutic drugs and/or nutritional compounds, such as salt solutions, and wherein the additional compounds are administered at a temperature Tb.

In embodiments thereof the first thermal means has at least a first portion and at least a second portion, wherein the at least first portion of the first thermal means is arranged to raise the body temperature of at least a first series of body parts of the individual to the target temperature Tb, and wherein the at least second portion of the first thermal means is arranged to prevent at least a second series of body parts to exceed a target temperature Ts, wherein the temperature Ts is lower than the temperature Tb.

Another aspect of the invention relates to, methods for subjecting an individual to a whole-body hyperthermia condition using a whole-body hyperthermia system as described above, the comprising the steps of
- applying means for monitoring the body temperature and/or vital signs of the individual;
- deriving from the monitoring of the body temperature an overall value, being the monitored body temperature Tm;
- generating a control signal from the monitored body temperature Tm for a first and second thermal means, for steering Tm to the target temperature Tb;
- raising the body temperature of the patient at a rate of at least 1 °C/hour using the first thermal means; and
- adjusting the patient's body temperature to the target temperature Tb using the second thermal means,
wherein the individual is kept in hyperthermia condition for at least 6 hours, or at least 8 hours, or at least 12 hours, during which period the monitored body temperature Tm of the patient does not deviate by more than 0.5 °C from the predetermined target temperature Tb.

In embodiments thereof the first and the second means differ from each other.

In embodiments thereof, the individual is kept in hyperthermia condition at a temperature between 40 and 42 °C.

In embodiments thereof, the individual is kept in hyperthermia condition for at least 12 hours.

In embodiments thereof, the individual is a cancer patient.

Another aspect of the invention are antitumor compounds for use in the treatment of cancer, wherein the compound is administered to a patient which is under a whole body hyperthermia of between 41.5 °C and 42.0 °C for at least 2 h.

The methods and devices of the present invention have the advantage that a whole body hyperthermia can be applied for a prolonged period and at narrowly controlled temperatures.

Similar to the external heating of blood via perfusion systems, embodiments of the present invention which rely on catheters take advantage of the blood stream of the individual to transport the heated blood throughout the entire body, including tissues and organs at the interior of the individual.

Embodiments of methods and devices of the present invention which use perfusions systems or the like have the advantage that a whole body hyperthermia can be obtained without physical damage to blood cells and organelles cause by peristaltic pumps. Indeed, prior art methods which rely on externally heating the blood require typically peristaltic pumps which damage blood cells and organelles.

In the methods and devices of the present invention, pumps such as a roller pump, a centrifugal pump, a pulsatile pump and a non-occlusive roller pump can be used for circulating blood with significantly less damage compared to a peristaltic pump.

In the present invention the combination of the external heating and the internal heating allow to bring the patient in a short time period to the desired temperature in the entire body without a risk of local overheating. Furthermore, the heating of the blood in combination with other means provides a homogeneous heating in the body (such as temperature controlled air supply, nutrient supply, ambient temperature, heat flux sensors). Monitoring of the temperature by e.g. internal temperate probes or heat sensitive cameras), allows to adjust the heat supply of a heating system.

### Brief Description of the Drawings

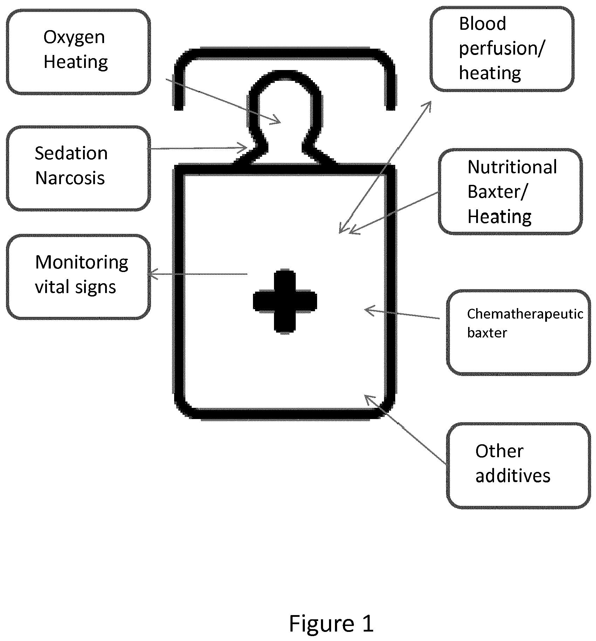

Figure 1 is a schematic illustration of the system according to the present invention.

### Detailed description of the invention

It is established that the temperature of the human body can be increased up to a temperature of 43 °C for a longer period without any adverse effects. However, some body parts cannot exceed this temperature. As a consequence, temperature T_{b} is in the range of 40.5 °C to 43 °C, preferably in the range of 41 °C to 42.5 °C, more preferably in the range of 41.5 °C to 42.0 C°. It is further understood that good results can be obtained when T_{b} is around 41.5 °C.

The whole-body hyperthermia system comprises typically a first thermal means and a second thermal means for the heating of the blood by extracorporeal or internal devices. The invention is further described with emphasis on those embodiments wherein two different thermal means are used. Nevertheless, as cited below, one single thermal means can be used in alternative embodiments.

The first thermal means are arranged for raising the patient's body temperature at a rate of at least 1 °C/hr, preferably at a rate of at least 2 °C/h, more preferably at a rate of at least 3 °C/h, even more preferably at a rate of at least 4 °C/h, more preferably at a rate of at least 5 °C/h, even more preferably at a rate of at least 6 °C/h and most preferably at a rate of at least 7 °C/h.

As mentioned herein above, some vulnerable body parts of the human body cannot be exposed to a temperature which exceeds a certain threshold value. For example, the temperature of the brain cannot exceed 43 °C as brain damage could occur.

First thermal means are generally provided with at least a first portion for heating a first series of body parts of the patient to the target temperature T_{b} and at least a second portion for preventing a second series of body parts of the patient of exceeding a temperature Tₛ, which is lower than T_{b}.

Preferably, Tₛ is not higher than 41.0 °C.

Hence, by avoiding the more vulnerable body parts, such as the brain, to exceed the temperature Tₛ, tissue damage of the vulnerable body parts can be avoided, while other body parts, such as the torso are allowed to be heated to the target temperature T_{b}.

In specific embodiments the head, or a part thereof is kept as a precaution at a lower temperature (e.g.) 0,5 °C, 1 °C, 2° C than the remainder of the body under hyperthermic condition, using e.g. a helmet with cooling or a gel containing blanket with cooling. In addition, pressure sensors can be provided to measure excess pressure in the skull which is indicative for overheating of the brain and subsequent brain damage.

It is understood that temperature control of all body parts at all times is a crucial aspect for hyperthermia treatment, so that upon heating, vulnerable body parts do not exceed a certain threshold temperature. It is understood that the first series of body parts and the second series of body parts do not overlap.

The methods and apparatus of the present invention envisages that the temperature of the body is initially raised to 42, or 42,5 or 43 °C for a period of about 15, 30, 45 or 60 minutes and afterwards kept at the desired target temperature of target temperature.

Equally, a local heating above the target temperature can be applied at a region of the body comprising a tumour. Such heating makes the tissue more vulnerable for a subsequent treatment such as local irradiation.

Typically, first thermal means may include a thermal bath, or a garment. A thermal bath may be divided in two parts, wherein a first part is arranged for heating the first series of body parts to the target temperature T_{b}, and a second part which is arranged for preventing the second series of body parts to exceed the lower temperature Tₛ. In order to raise the patient's body temperature, the bath may be filled with a substance such as heated water or molten wax. Alternatively, a garment may be carried by the patient or wrapped around the patient's body, and typically comprises a fluid circuit that provides a flow of heated fluid around the body.

According to one embodiment, the patient's body temperature is raised to the predetermined target temperature T_{b} by using heat radiation. For this purpose, the patient can be placed in a confined space, which may comprise isolating means for secluding the second series of body parts from overheating. Typically, the heat radiation means make use of infrared A radiation.

According to a preferred embodiment, the patient's body temperature is raised to the predetermined target temperature T_{b} making use of a closed loop fluid circuit.

Preferably, a so-called internally heated fluid circuit (internally heated fluid circuit", herein after) can be used, wherein the internally heated fluid circuit can comprise a flexible catheter, which is inserted into a blood vessel through the vascular system of the patient and wherein a fluid is circulated. The catheter further comprises a heat-transfer element which is positioned in the blood vessel, thus enabling the transfer of heat between the blood and the fluid of the fluid circuit. The fluid is pumped through the catheter of the closed circuit.

Alternatively, use can be made of a so-called externally heated fluid circuit (externally heated fluid circuit", herein after) wherein blood is withdrawn from the human body in a continuous mode, and wherein the blood is circulated in a continuous flow partially outside of the human body. The blood is heated outside of the human body in a heat-exchanger and subsequently returned to the body by a pump. As a consequence, the blood circulatory system of the human body is part of the closed loop fluid circuit.

In specific embodiments the natural breathing of an individual can be replaced by external devices. Alternatively, oxygen is supplied to the blood via the extracorporeal blood circulation devices.

### Advantage and shortcomings

### First Thermal Means

Advantageously, the first thermal means allow for a rapid, controlled heating of the patient's natural body temperature to a higher temperature. A rapid controlled heating has as an advantage that the overall treatment will take less time. This is both in the economic interest of the healthcare provider as advantageous for the comfort of the patient.

However, the first thermal means, arranged for providing a fast heating of the body temperature, would have to be adjusted quite early in the heating process in order to avoid overshooting of the target temperature T_{b}. It must be remembered that exceeding a threshold temperature has to be avoided at all times, due to the risk of causing permanent damage to vulnerable body parts and organs such as the brain. As a result, the patient's body temperature, although raised at a high speed in the beginning of the treatment, would only converge slowly to the target temperature T_{b}.

Overshooting can be avoided by providing PID controllers wherein the controller allows an initial fast heating and is stepwise adjusted to provide a slower heating followed by a steady state control to correct minor variations.

According to one embodiment of the present invention, the first thermal means are arranged for providing a fast heating of the patient's own body temperature to a temperature T_{b}-X, at a rate as determined here above. X is in the range of 0.2 °C to 2 °C, preferably in the range of 0.4 °C to 1.6 °C, more preferably in the range of 0.6 °C to 1.2 °C.

Fast heating by the first thermal means of the patient is typically performed by external heating of the blood system, and optionally combined with heating by a catheter.

Heat loss occurs via breathing and mainly via the skin. The fast heating is thus generally combined with means such as isolating blankets or gel packs, warm water baths, heated cabinets and the like. Also blankets and gel packs can be connected to a water supply to maintain the skin moist and thereby preventing dehydration.

Air used for respiration can be heated or alternatively by long term anaesthetics, the lung function is interrupted and oxygen supply to the blood is provided via an external blood circuit.

### Second thermal means

The apparatus of the whole-body hyperthermia treatment system according to the present invention further comprises a second thermal means, wherein the second thermal means comprise a fluid circuit which is at least partially within the patient, wherein a fluid, heated by a heat exchanger to a set temperature T_{f}, is circulated through the fluid circuit.

The temperature T_{f} is in the range of T_{b} +/- 0.5 °C. Preferably, the temperature T_{f} is equal to T_{b}.

According to one embodiment of the present invention, the fluid circuit is an externally heated fluid circuit, wherein the fluid is the blood of the patient and the patient's blood circulatory system is part of the fluid circuit. Part of the patient's blood is removed from the body, then heated by a heat-exchanger, external to the body, and subsequently returned to the body, where the heated blood is circulated further through the patient's body. This external circuit allows the adjust the oxygen content of the blood.

In a preferred embodiment according to the present invention, the fluid circuit is an internally heated fluid circuit, comprising a catheter wherein a heating fluid is circulated. The catheter is introduced in a blood vessel of the patient's body and a fluid is circulated through the catheter. The catheter further comprises a heat exchanger which is arranged for exchanging heat between the heating fluid and the patient's blood. The heating fluid is heated outside of the body in a heat exchanger. The heating fluid is typically circulated through the fluid by a pumping device.

Typically, the pumping device is a peristaltic pump, for heating during limited time periods. Perfusion pumps are used as an alternative since they cause less physical damage to the blood cells.

It has now been found that the combination of the first and second thermal means allow for effective heating of the patient's body.

Once a desired temperature is obtained using the first means, the first means is switched of and subsequent heating is performed by the second means. Alternatively or in addition, the heating by first means is lowered by lowering the heating or lowering the flow rate of heated blood from an external blood circuit.

In one embodiment according to the present invention, the whole-body hyperthermia system is further provided with means for monitoring the patient's body temperature and/or vital signs. The means for measuring vital signs include means for measuring the respiratory activity, if respiration is maintained, as well means for measuring the cardiac activity of the patient while under hyperthermia treatment.

The temperature of the patient, as well as the vital signs, are monitored using procedures well known in the art.

In one embodiment, the means for measuring the body temperature of the patient comprise at least one single temperature probe for monitoring at least one single temperature signal at at least one location on or within the patient.

In a preferred embodiment, the means for measuring the body temperature comprise at least two temperature probes. Preferably, at least one means for measuring the body temperature is located on the skin of the patient, thereby measuring the temperature of the skin of the patient, and at least one is located on a body part of the patient, other than the skin.

A further means for measuring the body temperature comprises a heat sensitive camera, which can measure the temperature of a significant surface of the skin.

In a more preferred embodiment according to the present invention, the means for monitoring the patient's body temperature comprise a plurality of temperature probes, which generate a plurality of temperature signals. Preferably, the temperature signals are measured in a continuous mode.

The whole-body hyperthermia system further comprises means for deriving, preferably in a continuous mode, from the at least one temperature signal an overall value, which is the monitored body temperature Tₘ. The measured body temperature Tₘ equals the one single temperature signal, when only one single temperature probe is used. When at least two temperatures probes are used, the measured body temperature is calculated from the at least two temperature signals.

The monitored body temperature Tₘ is derived from the at least two temperature signals according to procedures well known in the art.

The monitored body temperature Tₘ is derived in such a way that the optimal condition for hyperthermia treatment of the patient is considered to be achieved, when Tₘ is equal or close to the predetermined target temperature T_{b}.

It is hereby understood that the monitored body temperature Tₘ is considered equal or close to the predetermined target temperature T_{b}, when Tₘ is within the range of at most 0.5 °C, preferably of at most 0.4 °C, more preferably of at most 0.3 °C, even more preferably of at most 0.2 °C and most preferably of most 0.1 °C from the predetermined target temperature T_{b}.

In one embodiment according to the present invention, the whole-body hyperthermia system comprises a controller. The controller is arranged to use the measured body temperature Tₘ as an input signal for generating a control signal to steer the first and second thermal means, allowing thereby Tₘ to approach T_{b} and stabilize at this temperature.

For the purpose of the present invention, the above described first means and second means are typically different. In specific embodiments the first and second means can be the same, whereby one heating means is initially set to provide a fast heating, and subsequently set to obtain and maintain the target temperature.

This can be performed by a perfusion pump which has less mechanical impact on the physical integrity of the blood than a peristaltic pump.

In one embodiment according to the present invention, the whole-body hyperthermia system comprises means for bringing the patient under sedation or under anaesthesia.

For the purpose of the invention, the term "sedation" refers to the relaxed state of the patient, with or without loss of consciousness, after the administration of drugs. A patient under sedation can be synonymous for a patient under narcosis or under anaesthesia. The means for bringing the patient under sedation or under anaesthesia are provided to be supplied to the patient at a temperature T_{b}.

Specific embodiments refer to complete narcosis with no breathing. This has the advantage that heat loss via transpiration or via breathing is avoided.

Hereby the necessary oxygen supply for the body is provided by extracorporeal oxygenation of the blood. This is especially advantageous when blood is heated via an extracorporeal circuit.

In one embodiment according to the present invention, the whole-body hyperthermia system comprises means to supply oxygen to the patient. For the purpose of the invention, the term "oxygen" refers to any oxygen-containing gas that can be administered to a patient under sedation.

The oxygen supplying means are provided to supply oxygen to the patient at a temperature T_{b}. Advantageously, it has been found that the monitored body temperature Tₘ reaches the predetermined target temperature T_{b} at a faster pace. Furthermore, it has been found that supplying oxygen at the temperature T_{b}, allows the monitored body temperature Tₘ to stabilize in a simple way at or close to T_{b}.

Upon complete narcosis spontaneous breathing stops and oxygen is supplied via a mask. Typically, the temperature of the applied gas is adapted to avoid heat losses by introduction of cold gasses.

When an extracorporeal system is used to heat the blood of an individual, oxygen can be supplied to the blood of the patient, which allows to reduce of replace oxygen supply by a breathing apparatus.

In one embodiment according to the present invention, the whole-body hyperthermia system is arranged to raise the patient's body temperature to the target temperature T_{b} for at least a period of time tₚ. The period of time tₚ is at least 6h, typically at least 12 hours 12h, preferably at least 14h, more preferably at least 16h, even more preferably at least 18h, more preferably at least 20h, even more preferably at least 22h, and most preferably at least 24h.

The systems and methods of the present invention rely on an interaction of a network of temperature sensors, and a network of heat fluxes sensors for raising and maintaining the hyperthermic conditions in an individual. Controllers receive signals from the various sensors and steer the various devices, via feedback systems.

The main heat flux to the individual is provided via heating of the blood, with extracorporeal heating and/or internally via intravenous catheter.

Other heat fluxes comprise:
- The heating of the environment of the individual by temperature controlled air, fluids or gels. Compared to gels or fluids, the direct access to the skin has the advantage that the skin can be easily monitored and heated and isolated to prevent or counter heat loss. Local application of warm air or infrared radiation allows to heat those portions of the body with predominant. In addition to air, the individual might be placed on a heated table whereby typically temperature sensors are placed between the table and the body. The table is arranged to allow heating at specific parts of the table.
- The heating of oxygen comprising gasses during mechanic breathing. As indicated above, this can be partially or completely replaced by oxygen supply directly to the blood.
- The heating of fluids supplied to the individual such as nutrients, liquids for hydration, liquids with medicaments, or liquids with anaesthetics.
- The minimisation of heat losses by using isolated tubing with short length and from material with low heat capacity or by heating the air of the cabinet in which the patient is located.

Temperature sensors and heat flux sensors provide signals to controllers that can adjust the amount of heat that is transported to the patient. This can be done by adjusting the temperature of the liquids and gasses, but can also be achieved by adapting the flow rate of liquids and gasses.

Temperature sensing can be on the body by measuring the temperature within the body or at the outside of the body. Temperature sensing can be remote via heat sensitive cameras. Heat sensing can also be indirect by measuring the temperature of the air in the cabinet surrounding the patient, the temperature of the blood in an extracorporeal system when it leaves the body, the temperature of exhaled air, the temperature of urine.

The controllers typically act via feedback mechanisms using proportional-integral-derivative (PID) regulation to prevent overshooting to undesired and unacceptable temperatures.

Each device providing heat to the individual can have its own controller. Alternatively, a plurality of controllers or all controllers can be incorporated in one dedicated controller device.

The coordinated operation of the elements of the system allow a fast heating of the individual without overshooting and allow to maintain a desired temperature which is accurate over time and homogenous for the entire body.

In typical embodiments, the systems and methods of the present invention, the individual is placed in a sarcophagus like cabinet wherein all heating elements and controllers are outside the cabinet whereby the different liquids and gasses are provided to the individual via tubing. Alternatively, one or more heating elements are placed within the cabinet to shorten the distance between heated medium and the patient. This still allows to have parts of the system outside the cabinet. For examples gas bottles with air or oxygen air are placed outside the cabinet. "Cold" gas subsequently enters the cabinet where it is heated close to the individual.

In order to prevent dehydration of the body via the skin the cabinet can contain a sensor to monitor the humidity of the ambient air. Humidity can be adjusted by e.g. evaporating water in the cabinet, or by humidifying the gasses (air or oxygen) which enter the cabinet. The foil wrapped around the body is not only a means of heat isolation but can also be a means to prevent or reduce dehydration through the skin.

Other embodiments make use of larger cabinets, e.g. when the individual is placed in a bath with fluid, or specific larger apparatus is needed within the cabinet.

Advantageously, it has been found that the whole-body hyperthermia system according to the invention can be used for treating an individual who can benefit from a long term (i.e. at least 6 hours) hyperthermia treatment. As explained before, such treatment is beneficial for cancer since tumour cells are more sensitive for high temperatures. The method is also applicable for the treatment of diseases wherein under normal condition fever occurs. Accordingly, the method is also applicable to bacterial and viral infections. For example, the method can be used in the treatment of antibiotic resistant bacteria, or of viruses for which no, or no effective antiviral compound have been developed.

In one embodiment according to the present invention, the whole-body hyperthermia system is further provided with means for administering additional compounds to the individual. Non-limitative examples of the additional compounds include transfused blood, nutritional compounds such as salt solutions. Preferably, the means are arranged to administer chemotherapeutic compounds. The means for administering additional compounds are arranged so that the additional compounds are administered at a temperature T_{b}.

In the methods of the present invention different sensors and different types of tubing are used to maintain and adjust the hyperthermic condition and to monitor and control functioning of the body.

To facilitate the operation of the system of the present invention it is advantageous to provide a kit comprising tubing for a fluid circuit for blood supply to the patient and comprising sensors and wiring to monitor the temperature or heat loss from the patient.

A typical fluid circuit for arterial blood supply and venous blood drain, is an access device with 2 separate lumen (cannula) or a double lumen access device also called catheter. The fluid circuit is optionally foreseen with one or more selected from the group consisting of a blood pressure sensor, optical blood sensor, temperature sensor, air bubble monitor, air bubble trap, gas and or fluid flow meter [e.g. velocity], gas flow filter, oxygen supply, oxygen saturation meter, heparin and or other fluid/gas access points, membrane oxygenator, heat exchanger.

Optionally the fluid circuit is isolated against heat loss or blood degradation to prevent exposure to light.

In addition to the fluid circuit for arterial blood supply and venous blood drain, the kit contains one or more of a temperature sensor, a heat flux sensor, a sensor for measuring respiratory activity, a sensor for measuring cardiac activity.

The fluid circuit also allows to analyse blood inline within the fluid circuit or to withdraw blood for analysis. Parameters which can be determined are for example pH, salts, haematocrit values, content of oxygen and other gasses.

The disposable to be conceived in a 'cassette' like (pre-wired) form factor for easy handling and mounting on the device reducing the risk for confusion and / or wrong connections.

Additionally, the disposable can be foreseen with an insolation/isolation foil or suit for the patient. Additionally, the disposable can be foreseen with a hat-like cover to connect to a cooling device especially to cool the patient's brain.

The whole-body hyperthermia system is also useful for the treatment of collagen vascular diseases such as arthritis and psoriasis and for treating hypothermia.

Furthermore, when the system is used for hyperthermia treatment of cancer patients, it has been unexpectedly found that the rate of the temperature increase of the patient's body influences the annihilation mechanism of malign tumour cells. A fast heating rate promotes tumour cell destruction via cell necrosis, whereas a slower heating rate promotes cell destruction via apoptosis.

Since the hyperthermic conditions strongly decrease the viability of tumour cells, these cells become more susceptible to anticancer drugs, or irradiation. Lower doses of drugs or irradiation decrease side effects of these therapies.

Furthermore, the heating of the whole body not only has an effect on the primary tumour, localised in an organ of the body, but also on eventual circulating cells,

After heating, i.e. after the patient's body temperature has achieved the predetermined target temperature Tb, the first thermal means can be in principle removed from the patient and the temperature can be maintained by the second thermal means, and the patient can be wrapped in or covered by a heat barrier, such as a blanket, in accordance with procedures well known in the art.

In alternative embodiments one thermal means is used which first heats the individual to a desired temperature and afterwards maintains the patient at the desired temperature.

### Examples

### Example 1. in vitro model for optimization of the first means for heating.

A fibroblast cell line (Normal Human Dermal Fibroblasts (NHDF) and a tumour cell line are incubated in DMEM with fetal calf serum in an incubator with 5% CO₂. Suitable cell lines are for example Hela cells, HT-1080 (connective tissue celline with activated N-ras oncogene), A375 (malignant skin melanoma cell line), and A549 (adenocarcinomic human alveolar basal epithelial cells).

Cells are placed in the incubator at 37 °C, whereupon the temperature is raised to 41.5 °C over a time period ranging from 30 minutes to 4 hours. The volume of the medium on the cells is kept to a minimum to avoid delay in the heating.

Cells viability is determined and apoptosis and necrosis of cells is measured.

The experiment allows to determine the effect of a shallow or steep increase of the temperature as achieved by the first means when applied on the body.

### Example 2. in vitro model for determining efficiency and specificity.

The above described cells are incubated for different time periods (4, 6, 8, 12, 16, 20, 24, 36 hours) at different temperatures (37, 39, 40, 40,5, 41, 41.5 and 42 °C).

Cells viability is determined and apoptosis and necrosis of cells is measured.

The experiment allows determining the efficiency of the method (% killed tumour cells) and the specificity (%killed tumour cells versus killed normal cells).

The above system can be further used to mimic conditions in tumour tissue (hypoxia, pH) and to determine the effect on cells which have been irradiated or treated with cancer medicaments.

### Example 3. in vivo model for long term hyperthermia.

Mice with a tumour are placed under anaesthetics on a heated plate in a heated cabinet. For small laboratory animals the heat transfer is sufficient enough to provide a whole body hyperthermia without the application of first and second means as described in the specification.

Mice are killed and tumour tissue is examined for cell death.

The animal model allows comparing cell death as obtained in example 2 and in the mouse model using the same time period and the same temperature.

## Claims

1. A whole-body hyperthermia system for raising the body temperature of the entire body of an individual to a predetermined target temperature Tb and for stabilizing said body temperature at said target temperature Tb, said system comprising:
- a cabinet with isolation and heating for maintaining an individual at an elevated temperature,
- at least one thermal means for raising and stabilising the temperature of said individual,
- one or more devices to prevent or counter heat loss from the individual,
- one or more sensors monitoring the temperature or heat flux of the individual, connected to controllers steering said devices to prevent or counter heat loss and steering the at least one thermal means.

2. The system according to claim 1, wherein the devices to prevent or counter heat loss are devices for heating air and/or oxygen for respiration, or devices for heating liquids being introduced in the body.

3. The system according to claim 1 or 2, wherein the sensor monitoring the temperature of the individual are sensors measuring the temperature internally, sensors measuring the temperature externally and contacting the skin, or sensors measuring the temperature externally and not contacting the skin.

4. The system according to any one of claims 1 to 3, comprising a first thermal means for raising said body temperature at a rate of at least 1 °C/hour, and at least a second thermal means for further adjusting the body temperature to said target temperature Tb,
**characterised in that**,
said second thermal means comprise a fluid circuit, which is at least partially within the individual, wherein a fluid, heated by a heat exchanger to a set temperature Tf, is circulated through said fluid circuit.

5. The whole-body hyperthermia system according to claim 4, wherein said first and said second means are different from each other.

6. The whole-body hyperthermia system according to claim 4 or 5, wherein said fluid circuit is an internally heated catheter.

7. The whole-body hyperthermia system according to any one of claim 1 to 6, wherein said system is provided with means for monitoring the body temperature and/or vital signs of the individual, such as respiratory activity, cardiac activity and blood parameters.

8. The whole-body hyperthermia system according to any one of claims 1 to 7,
wherein
said system is provided with means to administer additional compounds to the individual, such as transfused blood, chemotherapeutic drugs and/or nutritional compounds, such as salt solutions, and wherein said additional compounds are administered at a temperature Tb,
and/or
wherein said system is provided with one or more isolation elements for an individual, such as isolation foil or suit for torso or limbs, or a helmet or cap like device for application on the head.

9. A disposable kit for use in the system according to any one of claims 1 to 8, comprising tubing for a fluid circuit for blood supply to an individual and comprising sensors to monitor the temperature or heat loss from the patient.

10. A method for subjecting an individual to a whole-body hyperthermia condition using the whole-body hyperthermia system according to any one of claims 1 to 9, the method comprising the steps of:
- placing the individual in a heated cabinet.
- applying means for monitoring the body temperature
- deriving from said monitoring of the body temperature an overall value, being the monitored body temperature Tm;
- generating a control signal from said monitored body temperature Tm for thermal means, for steering Tm to the target temperature Tb;
**characterized in that**,
the individual is kept in hyperthermia condition for at least 6 hours, or at least 8 hours, or at least 12 hours, during which period the monitored body temperature Tm of said patient does not deviate by more than 0.5 °C from said predetermined target temperature Tb.

11. The method according to claim 10, wherein heat losses from the individual are compensated by one or more of the following steps:
- heating the skin of the individual or parts thereof by controlling the temperature of the air surrounding the individual;
- applying isolation on one or more parts of the body;
- heating air used for respiration to a controlled temperature;
- heating liquids for administering, nutrients, moisture or medicaments to a controlled temperature.

12. The method according to claim 10 or 11, wherein said first and said second thermal means are used which differ from each other.

13. The method according to any one of claims 10 to 12, wherein the individual is kept in hyperthermia condition at a temperature between 40 and 42 °C and/or wherein the individual is kept in hyperthermia condition for at least 12 hours.

14. Use of a kit in accordance with claim 9 in a method according to any one of claims 10 to 13.

15. An antitumor compound for use in the treatment of cancer, wherein the compound is administered to a patient which is under a whole body hyperthermia of between 41.5 °C and 42.0 °C for at least 6 h.
